# EUROPEAN PATENT APPLICATION

(11) **EP 3 501 433 A1**
(43) Date of publication of application: **26.06.2019**
(21) Application number: 17209279.3
(22) Date of filing: 21.12.2017
(51) Int. Cl.: A61B 17/30, A61F 9/007, A61B 17/04

(54) **APPARATUS FOR HANDLING A ROD SHAPED ELEMENT TO BE INSERTED INTO OR REMOVED FROM THE EYE OF A PATIENT**

(71) Applicant: Universiteit Maastricht, 6211 LK Maastricht (NL); Academisch Ziekenhuis Maastricht, 6229 HX Maastricht (NL)
(72) Inventor: BERTENS, Christian Johannes Franciscus, 6223 AW Maastricht (NL); GIJS, Marlies, BE-2480 Dessel (BE); NUIJTS, Rudolph Matheus Marie Antonius, 6255 AM Noorbeek (NL)
(74) Representative: Algemeen Octrooi- en Merkenbureau B.V.

(57) **Abstract**

The invention relates to an apparatus for handling a rod shaped element to be inserted into or removed from an eye of a patient, the apparatus comprising tweezers having a pair of legs joined to one another at a first end, and a second end of each leg comprises a gripping element, wherein the gripping elements can be manually moved from a first spaced apart position to a second gripping position.

The invention also relates to a system comprising such an apparatus and to the use of such an apparatus.

## Description

The invention relates to an apparatus for manually handling a rod shaped element to be inserted into or removed from an eye of a patient, the apparatus comprising tweezers having a pair of legs joined to one another at a first end, and a second end of each leg comprises a gripping element, wherein the gripping elements can be manually moved from a first spaced apart position to a second gripping position.

The invention also relates to a system comprising such an apparatus and to the use of such an apparatus.

Tweezers for handling different kind of objects are known. A drawback of the known tweezers is that these are not configured for inserting or removing rod shaped objects/elements into or from an eye of a patient. In addition, the known tweezers may injure the eye of a patient during insertion or removal of the rod shaped objects. The forces to be exerted on the objects to be inserted in the eye of a patient by the known tweezers during pickup and/or holding of the object are relatively uncontrolled, such that the objects can be easily broken or damaged during pickup, insertion, and/or holding of the object by the known tweezers.

Therefore, it is an object of the present invention to provide an apparatus for picking up a rod shaped element to be inserted into or removed from the eye of a patient in a controlled manner to minimize the risk of injuring the eye and/or damaging the rod shaped element.

This object is achieved with an apparatus as defined in claim 1.

The apparatus comprises tweezers having a pair of legs joined to one another at a first end, and a second end of each leg comprises a gripping element. The gripping elements can be manually moved from a first spaced apart position to a second gripping position. The gripping elements are configured to pick up and hold in the second gripping position the rod shaped element between its longitudinally opposing ends. In other words, the gripping elements are designed to pick-up, hold and release the rod shaped element in a predetermined orientation with respect to the gripping elements of the tweezers. In fact, the gripping elements pick-up and hold the rod shaped element at least partially around its outer longitudinal wall or walls between two longitudinally opposing ends of the rod shaped element. Further, by means of the tweezers it is possible to pick up / remove the rod shaped element, for example from the eye of a patient, or to insert the rod shaped element into the eye of a patient in a controlled manner. For facilitating picking up / removing / inserting the rod shaped element by means of the gripping elements the second end of each leg of the tweezer is beveled at a predetermined angle between 10-30 degrees from a normal plane to a longitudinal axis of the apparatus.

The rod shaped element may be without any longitudinal extending edges/corners, e.g. cylindrical-shaped, or be provided with longitudinally extending edges/corners between the longitudinally opposing ends, e.g. cuboid-shaped. It may also be possible to use a combination of these two shapes in the length direction of the rod shaped element.

The word "rod" is used herein to denote that the length dimension of the rod shaped element is the largest dimension. The length is larger than the dimensions defining the surface area of longitudinal cross sections of the rod shaped element, i.e. cross sections that are perpendicular to the longitudinal axis or centre axis of the rod shaped element. The length of the rod shaped element may be 5-25 mm, more preferred 10-20 mm. The distance between the longitudinal axis and an outer wall of the rod shaped element extending between the two longitudinally opposing ends is typically between 0,1-2,0 mm, more preferred 0,2-0,4 mm.

As the dimensions of the rod shaped element to be inserted in the eye of a patient are relatively small, it is relatively difficult, even for medically trained professionals, to pick-up such an element. Hence, for facilitating take up of the rod shaped element by the gripping elements, the second end of each leg of the tweezer is beveled at a predetermined angle between 10-30 degrees from a normal plane to a longitudinal axis of the apparatus. The predetermined angle is preferably 15-25 degrees, or even more preferred 15-20 degrees. This angle of the second end of the legs forming the gripping elements of the tweezer reduces the risk of damaging the rod shaped element during pick-up, because this configuration provides a user more control over the gripping elements and/or a better overview during the pick up or insertion process. This is in particular a relevant aspect if the rod shaped element is an eye medicating construction, such as an ocular coil, to be inserted in the lower fornix of the eye. The ocular coil may be a hollow wire which may be covered with a helical coil and/or a coating on the outer wall of the wire, wherein the lumen of the hollow wire may be filled with a drug, e.g. a drug eluting matrix. Such a drug eluting matrix is able to provide a constant drug delivery into an eye of a patient. The conventional tweezers may for example press the windings of the ocular coil open during take up which could damage the shape of the coil resulting into loss of the drug or even an incorrect drug delivery into the eye of the patient.

In a further aspect, the gripping elements are designed to surround more than 50% of the circumference of the rod shaped element around its central axis in the second gripping position of the gripping elements, preferably the gripping elements are shaped to surround more than 60% of the circumference of the rod shaped element around its central axis in the second gripping position, even more preferred more than 80% of the circumference of the rod shaped element around its central axis. Such a design of the gripping elements ensures that the rod shaped element can be stably hold between the gripping elements with a more uniform force distribution. Further, this configuration of the gripping element to surround more than 50 % of the circumference of the rod shaped element reduces the risk that the element is displaced between the gripping elements during picking up and holding. In this way the medical practitioners have a maximum degree of freedom during displacement of the rod shaped element between a pickup location and a final destination. In addition, the more uniform holding force distribution around the central axis ensures that the risk of damaging the rod shaped element is minimized.

The gripping elements may be configured to surround the circumference of the rod shaped element completely, i.e. 100%, or almost completely, i.e. more than 95%, such that a maximum uniform holding force distribution can be achieved around the central axis of the rod shaped element.

The gripping elements may have gripping surfaces oriented towards each other. These gripping surfaces can be manually brought towards each other in an at least partial contact or close to each other for holding and at least partially surrounding the rod shaped element. The gripping surfaces or at least one of the gripping surfaces may comprise at least one recess to receive at least a portion of the rod shaped element. In the recess a portion of the rod shaped element can be received such that the recess contributes in preventing that the rod shaped element is able to move in undesired directions. The shape of the recess may correspond to the shape of the portion of the rod shaped element to be received in the recess. If both gripping surfaces comprise a recess, these recesses may cooperate for gripping and holding the rod shaped element around its perimeter. This even further contributes in preventing that the rod shaped element is able to move in undesired directions, but also facilitates to surround the circumference of the rod shaped element by the gripping elements to stably hold the rod shaped element and to provide a more uniform holding force distribution around the central axis of the rod shaped element. Further, it is advantageous if the center line of a space for receiving a rod shaped element defined by the recess or by cooperating recesses when the gripping elements are in the second gripping position, extends at a predetermined angle between 10-30 degrees from a normal plane to a longitudinal axis of the apparatus, preferably the predetermined angle is 15-25 degrees, more preferred 15-20 degrees. The length of a recess measured in the direction of the centre line of the hole is smaller than the length of the rod shaped element.

In a further aspect, the gripping surfaces may be chamfered with respect to the longitudinal axis of the apparatus to restrict a maximum gripping force to be brought by means of the gripping elements on the rod shaped element. A chamfer angle of the chamfered area with respect to a virtual line extending parallel to the centre line of the tweezer or parallel to the longitudinal axis of the apparatus is smaller than 15 degrees, preferably 1-5 degrees. By means of the chamfered gripping surface(s) it is possible to hold the rod shaped element in a reliable manner by means of the gripping elements and at the same time the maximum force exerted on the gripping elements by means of a hand of an operator is restricted such that the risk that a person picking up and/or holding the rod shaped element exerts a force damaging the rod shaped element is minimized or even excluded.

In a further aspect of the apparatus, each leg of the tweezers may have a shape such that, when the apparatus is positioned on a flat surface, for example a table, a bottom surface of the first end is in contact with the flat surface, there is a distance between the flat surface and the second ends of the legs, preferably the distance between the lowest point of the second end of each leg and the flat surface is greater than the distance between the flat surface and top surface of the first end. Such a design of the legs further facilitates the pick up or the insertion process of a rod shaped element.

In another aspect of the apparatus, each leg of the tweezers may have a first portion located closer to the first end and a second portion located closer to the second end, wherein, when the gripping elements are in the first spaced apart position, the first portions of the legs extend parallel to each other, and the second portion of the legs have an orientation such that the distance between the legs decreases towards the second ends of the legs. This configuration of the legs improves the operation of the apparatus, in particular the pick up or insertion process of a rod shaped element to be inserted in the eye of a patient

In addition, each leg of the tweezer may have a curved design between the first end and the second end of the leg, wherein the curvature/curvatures is/are located closer to the second end than to the first end of the leg. The curvature(s) of the legs facilitate the pickup and holding process of the rod shaped element as described above, but also help to improve the overview for a user operating the apparatus for handling a rod shaped element with respect to an eye of a patient.

The apparatus may be from a material which does not damage a delicate/fragile rod shaped element such as a medicating construction and that minimizes the risk of injuring the eye of a patient. The apparatus may be made of a polymer such as polypropylene or nylon and the apparatus may be produced by injection molding or 3D printing. The second end of each leg comprises rounded edges to avoid injuring the eye during insertion or removal of the rod shaped element in or from the eye.

Further, the apparatus may comprise a grip to improve handling the apparatus during use. The legs may also be connected to each other between the first end and the second end of each leg. In still another aspect the apparatus can be produced by relatively low manufacturing costs. Further, the apparatus may be individually packed, sterile and/or disposable.

The invention further relates to a system comprising an apparatus as described above and a rod shaped element, wherein the rod shaped element has a first end and a longitudinally opposing second end and a central axis extending between the first end and the longitudinally opposing second end, preferably the rod shaped element is a medical construction such as an ocular coil as described above.

The invention further includes the use of the above described apparatus for handling a rod shaped element.

Same or similar advantages which have been already mentioned with respect to the apparatus apply to the system and/or the use of the apparatus in a same or similar way and therefore, these advantages will not be repeated.

The invention will now be explained in more detail with reference to an exemplary embodiment of the apparatus shown in the appended figures, in which:
Figure 1 shows a perspective view of an apparatus for handling a rod shaped element to be inserted into or removed from the eye of a patient;
Figure 2 shows an aspect of the apparatus shown in figure 1 in more detail;
Figure 3 shows a side view of the apparatus shown in figure 1;
Figure 4 shows a top view of the apparatus shown in figure 1;
Figure 5 shows a further aspect of the apparatus shown in figure 1 in more detail;
Figures 6A, 6B show another aspect of the apparatus shown in figure 1 in more detail
Figures 7A, 7B show photos of a rod shaped element such as an ocular coil to be inserted in an eye as shown in photo 7b.

Like parts are indicated by the same signs/numerals in the various figures.

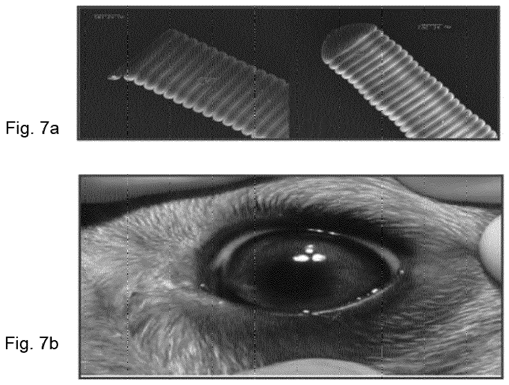

The figures shows various views and aspects of an apparatus 1 for handling a rod shaped element (see figure 7a) to be inserted into or removed from the eye of a patient. The patient may be human or non-human, such that the rod shaped element (see figure 7a) can be inserted into or removed from the eye of a human or an animal by the apparatus 1, preferably the animal is a mammal.

The rod shaped element shown in figure 7a is an eye medicating construction, more specific a cylindrical ocular coil. The ocular coil is a hollow wire which is covered with a helical coil, wherein the lumen of the hollow wire is filled with a drug such as a drug eluting matrix. This drug eluting matrix is able to provide a constant drug delivery into an eye of a patient. The ocular coil is inserted in the lower fornix of the eye and the drug is for example a drug that can be released into an eye of a person in need of a treatment. The length of the ocular coil may vary between 5-25 mm, more preferred 10-20 mm. The distance between the longitudinal axis and an outer wall of the ocular coil extending between the two longitudinally opposing ends is typically between 0,1-2,0 mm, more preferred 0,2-,4 mm. In other words the dimensions of the ocular coil to be inserted in the eye of a patient are relatively small. Hence, it is relatively difficult, even for medically trained professionals, to pick-up and hold such an ocular coil.

The apparatus 1 as shown in the figures is designed and configured to facilitate the pickup- or insertion-process of the above described ocular coil. The apparatus 1 is made of polypropylene or nylon and the apparatus may be produced by injection molding or 3D printing.

The apparatus 1 comprises a grip 3 and tweezers 5 having a pair of legs 7, 9 joined to one another at a first end 11, and a second end 13, 15 of each leg 7, 9 comprises a gripping element / a gripping portion 17, 19. In the exemplary embodiment of the apparatus 1 shown in the figures, the second end 13, 15 of each leg 7, 9 is designed as a gripping element 17, 19.

To reduce the weight of the apparatus and/or to reduce the material to be used to produce the apparatus 1, the apparatus 1 may be provided with openings 20 in the grip 3 and in legs 7, 9 as shown in the figures.

The gripping elements 17, 19 comprise gripping surfaces 21, 23 which can be manually brought in contact with each other or close to each other for holding the ocular coil. Each gripping surface 21, 23 comprises a recess 25, 27 to receive at least a portion of the ocular coil, wherein the recesses 25, 27 cooperate for surrounding and holding the ocular coil.

The second ends 13, 15 of the leg 7, 9 further comprise rounded edges to avoid injuring the eye during insertion or removal of the ocular coil in or from the eye (as shown in figure 7B).

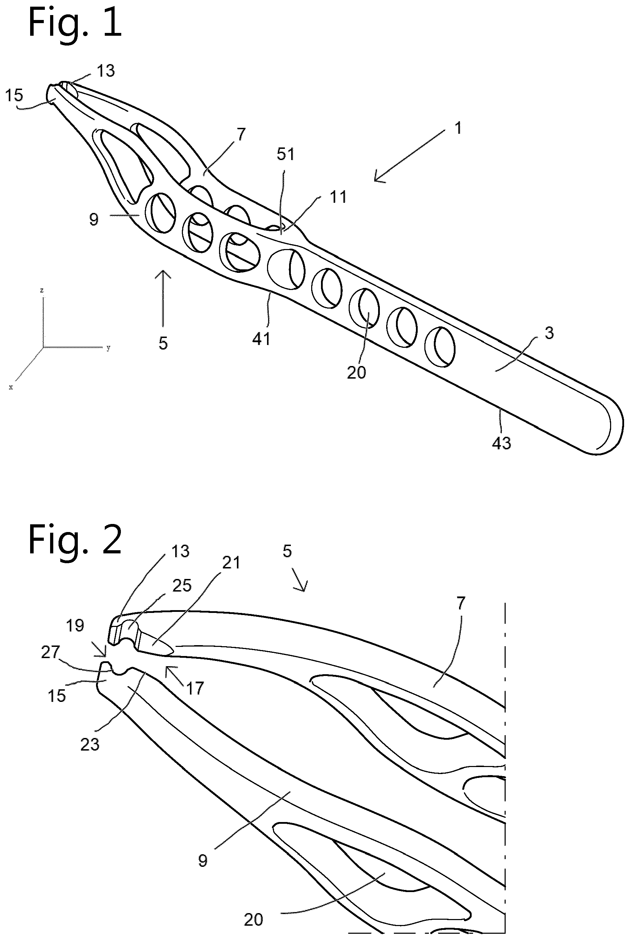

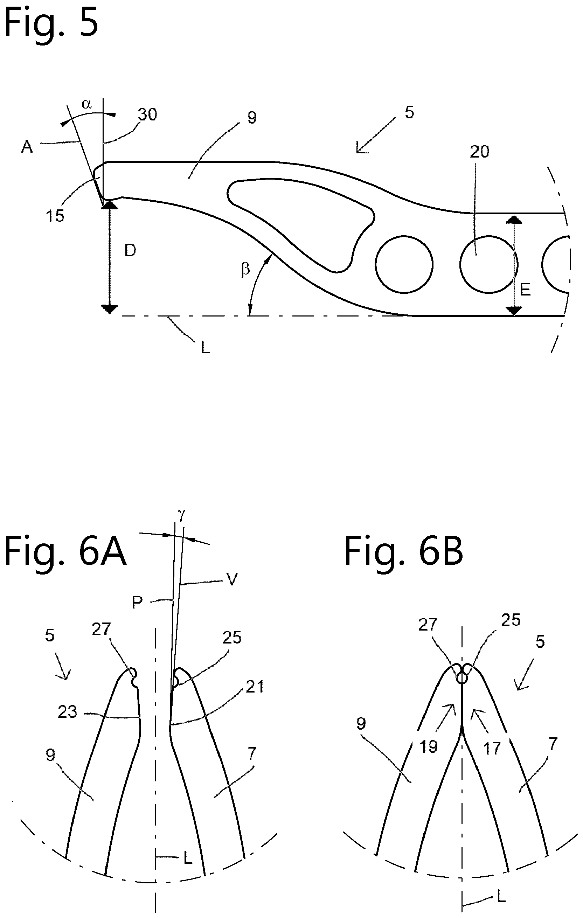

As shown in figures 6A and 6b the gripping elements 17, 19 can be manually moved by a user of the apparatus from a first spaced apart position as shown in figure 6A (also shown in figures 1, 2 and 4) to a second gripping position as shown in figure 6B.

The gripping elements 17, 19 are designed to pick-up, hold and release the ocular coil in a predetermined orientation with respect to the gripping elements, i.e. around its outer longitudinal wall(s) between two longitudinally opposing ends of the ocular coil. In the second gripping position as shown in figure 6B the cylindrical ocular coil has a cross section shape, i.e. circular, corresponding with the cross section shape, i.e. circular, of the two cooperating recesses 25, 27, wherein the recesses 25, 27 together have a diameter surrounding the circumference of ocular coil completely, i.e. 100%, or almost completely, i.e. more than 95%.

As shown in figure 5 the second end 13, 15 of each leg of the tweezers is beveled at a predetermined angle α of 20 degrees from a normal plane 30 to a longitudinal axis L of the apparatus for facilitating picking up the ocular coil and/or insertion of the ocular coil. Without wishing to be bound by theory, the present inventors have found out that a predetermined angle α (see figure 5) between 10-30 degrees preferably 15-25 degrees, more preferred between 15-20 degrees, results in a drastic improved of the functionality of the tweezers of the apparatus. For example, the overview for a user picking up an ocular coil is improved. Also, the picking up process of the ocular coil is much more simpler such that the operator needs no or minimal practice to pick up the ocular coil with the tweezers 5 of the apparatus 1. In addition, this angle α helps in reducing and/or controlling the forces exerted on the ocular coil during handling, in particular during the pick-up process. These advantageous aspects minimize the risk that an ocular coil is damaged during handling, e.g. the pick-up process, and also reduce the risk that an eye of a patient gets injured.

The recesses 25, 27 in the gripping elements 17, 19 define a hole or space for receiving an ocular coil. The hole or space shown in figure 6B is for example formed by two cooperating recesses 25, 27 and the hole extends in a direction from the plane of the drawing of figure 6B. The center line of the hole extends in the same direction as the beveled second end 13, 15 of the legs 7, 9, i.e. in a parallel direction indicated by the virtual line A in figure 5. Hence, the center line of the hole or space for receiving or holding an ocular coil extends at a predetermined angle of 20 degrees from a normal plane 30 to a longitudinal axis L of the apparatus 1. The length of recess 25, 27 measured in the direction indicated by line A, i.e. in a direction perpendicular to a cross section of the recesses 25, 27 as shown in figures 6A and 6B, is smaller than the length of the ocular coil.

The gripping surfaces 21, 23 facing each other are chamfered with respect to the a plane P extending parallel to longitudinal axis L of the apparatus 1 to restrict a maximum gripping force on the ocular coil in the second gripping position. As shown in figure 6A the angle γ between plane P and the plane V corresponding to the gripping surfaces 21, 23 is 1-3 degrees. The chamfer angle γ is maximally 15 degrees.

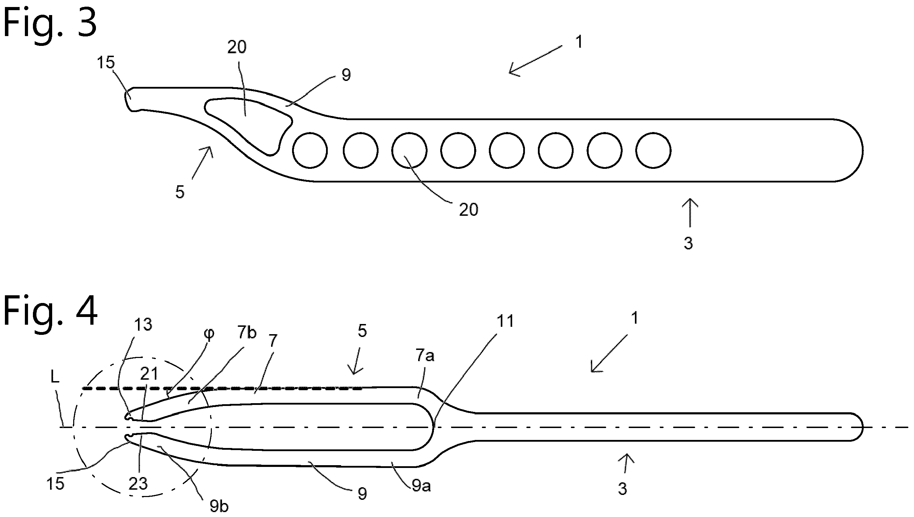

As shown in the figures 1, 3 and 5 the legs 7, 9 have a curved shape between the first end 11 and the second ends 13, 15 of the legs 7, 9. By means of the curvatures the functionality of the apparatus and operating the apparatus to pick up and introduce an ocular coil in the eye of a patient is further improved. Each leg 6, 7 has two curvatures, one curvature in a vertical direction (z-direction, see figure 1) as indicated with angle β in figure 5 and one curvature in a horizontal direction (x,y-direction see figure 1) as indicated with angle ϕ in figure 4. These curvatures in each leg 7, 9 are located closer to the second ends 13, 15 of the legs 7, 9 than to the first end 11.

By the first curvature identified with angle β in figure 5 each leg has a shape such that, when the apparatus is positioned on a flat surface for example represented by the longitudinal axis L (see for example figure 5 or defined by plane x-y as shown figure 1), a bottom surface 41 of the first end 11 and a bottom surface 43 of the grip 3 are in contact with the flat surface (L), and a distance D (see figure 5) is provided between the flat surface and the second ends 15 of the legs 5, 7. The distance D between the lowest point of the second end 15 of each leg 6, 7 and the flat surface L is greater than the distance E (see figure 5) between the flat surface L and top surface 51 of the first end 11.

By the second curvature identified with angle ϕ in figure 4 each leg 6, 7 of the tweezers 5 may have a first portion 7a, 9a (figure 4) located closer to the first end 11 and a second portion 7b, 9b located closer to the second end 13, 15 of each leg 6, 7. When the gripping elements 17, 19 are in the first spaced apart position as shown in figure 4, the first portions 7a, 9a of the legs extend parallel to each other, and the second portion 7b, 9b of the legs have an orientation such that the distance between the legs decreases towards the second ends 13, 15 of the legs.

The apparatus 1 as shown and described herein may be individually packed, sterile and/or disposable.

The legs 7, 9 may also be connected to each other (not shown) between the first end 11 and the second end 13, 15 of each leg 7, 9.

The ocular coil shown in figure 7a is relatively fragile. The ocular coil has a central axis extending between its longitudinally opposing ends. The gripping elements 17, 19 may have a different design as shown in the figures, i.e. the gripping elements may be designed to surround more than 50% of the circumference of the ocular coil around its central axis in the second gripping position of the gripping elements, preferably the gripping elements are shaped to surround more than 60% of the circumference of the ocular coil around its central axis in the second gripping position, even more preferred more than 80% of the circumference of the ocular coil around its central axis. Such a design of the gripping elements makes sure that the ocular coil can be stably hold between the gripping elements with a more uniform force distribution which further ensures that the risk of damaging the ocular coil is minimized.

Method for picking up, holding and/or releasing by means of an apparatus described herein or a tweezers of the apparatus described herein, a rod shaped element, preferably an eye medicating construction such as an ocular coil as described above. In the method the apparatus as described herein is used to pick-up the rod shaped element from a sterile holder. Then, the rod shaped element is hold and moved by the apparatus towards an eye of a patient. Finally, the rod shaped element is inserted into the eye of a patient by the apparatus. After a predetermined time period, for example a period from 4 hours to 4 weeks, the rod shaped element is removed for the eye by the apparatus. This method is in particularly beneficial to be used for inserting the rod shaped element into an eye of a person in need of a treatment, wherein the rod shaped element is able to release a drug for the above specified period in the eye of the patient.

## Claims

1. An apparatus for manually handling a rod shaped element to be inserted into or removed from an eye of a patient, the apparatus comprising tweezers having a pair of legs joined to one another at a first end, and a second end of each leg comprises a gripping element, wherein the gripping elements can be manually moved from a first spaced apart position to a second gripping position, wherein the gripping elements are configured to pick up and hold in the second gripping position the rod shaped element between its longitudinally opposing ends, wherein the second end of each leg of the tweezers is beveled at a predetermined angle between 10-30 degrees from a normal plane to a longitudinal axis of the apparatus for facilitating picking up the rod shaped element and/or insertion of the rod shaped element.

2. The apparatus according to claim 1, wherein the predetermined angle is 15-25 degrees, preferable 15-20 degrees.

3. The apparatus according to claim 1 or 2, wherein the rod shaped element is an eye medicating construction to be inserted in the lower fornix of the eye, preferably the rod shaped element is an ocular coil.

4. The apparatus according to any preceding claim, wherein the rod shaped element has a central axis extending between its longitudinally opposing ends, wherein the gripping elements are designed to surround more than 50% of the circumference of the rod shaped element around its central axis in the second gripping position of the gripping elements, preferably the gripping elements are shaped to surround more than 60% of the circumference of the rod shaped element around its central axis in the second gripping position, even more preferred more than 80% of the circumference of the rod shaped element around its central axis.

5. The apparatus according to any preceding claim, wherein gripping surfaces of the gripping elements to be brought in contact or close to each other for holding the rod shaped element comprise at least one recess to receive at least a portion of the rod shaped element, preferably each gripping surface comprises a recess which cooperates with the recess of the opposite gipping surface for holding the rod shaped element.

6. The apparatus according to claim 5, wherein the gripping surfaces facing each other are chamfered with respect to the longitudinal axis of the apparatus to restrict a maximum gripping force on the rod shaped element in the second gripping position.

7. The apparatus according to claim 6, wherein a chamfer angle is smaller than 15 degrees, preferably 1-15 degrees.

8. The apparatus according to claim 5, 6, 7 wherein the center line of a space for receiving a rod shaped element defined by the recess or by cooperating recesses when the gripping elements are in the second gripping position, extends at a predetermined angle between 10-30 degrees from a normal plane to a longitudinal axis of the apparatus, preferably the predetermined angle is 15-25 degrees, more preferred 15-20 degrees.

9. The apparatus according to any preceding claim, wherein each leg has a shape such that, when the apparatus is positioned on a flat surface, for example a table, a bottom surface of the first end is in contact with the flat surface, there is a distance between the flat surface and the second ends of the legs, preferably the distance between the lowest point of the second end of each leg and the flat surface is greater than the distance between the flat surface and top surface of the first end.

10. The apparatus according to any preceding claim, wherein each leg of the tweezers may have a first portion located closer to the first end and a second portion located closer to the second end of each leg, wherein, when the gripping elements are in the first spaced apart position, the first portions of the legs extend parallel to each other, and the second portion of the legs have an orientation such that the distance between the legs decreases towards the second ends of the legs.

11. The apparatus according to any preceding claim, wherein the legs have a curved shape between the first end and the second ends of the legs, preferably the at least one curvature is located closer to the second ends of the legs than to the first end.

12. The apparatus according to any preceding claim, wherein the second end of each leg comprises rounded edges to avoid injuring the eye during insertion or removal of the rod shaped element in or from the eye.

13. The apparatus according to any preceding claim, wherein the apparatus is a disposable apparatus and/or the apparatus is produced by injection molding or 3D printing, preferably the apparatus is made of a polymer such as polypropylene or nylon.

14. System comprising an apparatus as claimed in any of the preceding claims and a rod shaped element, wherein the rod shaped element has a first end and a longitudinally opposing second end and a central axis extending between the first end and the longitudinally opposing second end, preferably the rod shaped element is a medical construction, for example an ocular coil.

15. Use of an apparatus according to any of claims 1-13 for picking up and holding a rod shaped element.
